# DEMANDE DE BREVET EUROPEEN

(11) **EP 0 685 211 A1**
(43) Date de publication de la demande: **06.12.1995**
(21) Numéro de dépôt: 95401232.4
(22) Date de dépôt: 26.05.1995
(51) Int. Cl.: A61F 7/00, A61B 8/08, A61B 19/00

(54) **Utilisation d'un échographe en mode A pour la surveillance de la position d'un patient pendant une séance de thérapie, et procédé en comportant application**

(30) Priorité: 30.05.1994 FR 9406539
(71) Demandeur: TECHNOMED MEDICAL SYSTEMS, 69120 Vaux-en-Velin (FR); INSTITUT NATIONAL DE LA SANTE ET DE LA RECHERCHE MEDICALE (INSERM), F-75654 Paris Cédex 13 (FR)
(72) Inventeur: Chapelon, Jean-Yves, F-69100 Villeurbanne (FR); Blanc, Emmanuel, F-69230 St Genis Laval (FR)
(74) Mandataire: Hirsch, Marc-Roger

(57) **Abrégé**

L'invention concerne l'utilisation d'un échographe en mode A pour la surveillance de la position d'un patient pendant une séance de thérapie, ainsi qu'un procédé et un appareil en comportant application.

L'appareil de thérapie comprend un dispositif de thérapie (12) comprenant au moins un transducteur ultrasonore de thérapie (14) et au moins un transducteur ultrasonore de surveillance (16), caractérisé en ce que le transducteur ultrasonore de surveillance est un transducteur ultrasonore en mode A faisant partie d'un échographe en mode A, ledit appareil comprenant également des moyens (24, 28) pour faire émettre un signal court au transducteur ultrasonore en mode A, pour recevoir les échos de ce signal sur ledit transducteur en mode A, des moyens de comparaison (28) de l'écho reçu par rapport à un écho de référence et des moyens (28) de transmission du résultat de cette comparaison à un dispositif de commande (40).

La surveillance de la thérapie peut être automatique.

## Description

L'invention concerne essentiellement l'utilisation d'un échographe en mode A pour la surveillance de la position d'un patient pendant une séance de thérapie, ainsi qu'un procédé et un appareil en comportant application.

On sait que les ultrasons focalisés peuvent être utilisés pour le traitement de tumeurs. Généralement, on procède en plusieurs étapes, à savoir tout d'abord le repérage du volume à traiter, ensuite le calcul des positions de tir et enfin la réalisation de tirs successifs sur les positions calculées.

Suivant les méthodes connues, le tir est effectué soit "en aveugle", soit encore la période d'attente entre les tirs est mise à profit pour effectuer une image échographique de contrôle.

Le traitement "en aveugle" peut être dangereux car les séances sont souvent longues et le patient - même anesthésié - est susceptible de bouger. Dans ce cas, l'appareil risque d'endommager des parties saines ou même vitales.

Ainsi, en effectuant une image, on peut surveiller la position réelle du transducteur de tir par rapport aux organes avoisinant la cible. Cependant, cette méthode n'offre pas une sécurité absolue car on ne peut pas être certain que le personnel soignant portera une attention soutenue à l'écran de l'échographe et ce pendant toute la durée du traitement. Pour pallier cet inconvénient, il est possible d'utiliser les techniques d'analyse d'image mais celles-ci demandent une électronique et un logiciel qui sont spécifiques et complexes, ce qui augmente le coût de l'appareil et peut générer des pannes supplémentaires.

Un autre inconvénient de cette solution réside dans l'échographe de surveillance lui-même. Celui-ci peut être classique en ce sens qu'il comporte une tête ultrasonore à balayage comme décrit dans le document WO 92/15253. Dans ce document, il a été décrit l'incorporation d'une tête échographique à une sonde de thérapie, mais dans ce cas cette tête masque une partie du transducteur de tir, en réduisant ainsi l'efficacité de l'appareil comme cela est bien mentionné dans ce document où il est indiqué que l'on recherche toujours à maximaliser la surface d'émission du transducteur de tir.

On peut aussi obtenir une image échographique de la cible et de son voisinage sans réduire la surface d'émission du transducteur de tir. Pour cela, on utilise un élément du transducteur et on le connecte à un échographe de type "B" comme décrit dans la Revue IEEE 1992, Ultrasonics Symposium Proceedings, par Sanghvi N. T. Foster, R.S., Fry FJ, Birhle R., Hennige C. and Hennige L.V. dans l'article pour titre "Ultrasound intracavitary system for imaging, therapy and planning, and treatment of focal diseases", pages 1 249 à 1 253 A chaque position de la tête de tir, une ligne de l'image sera obtenue. Pour obtenir l'image entière, il faut alors déplacer la tête de tir séquentiellement. Cette procédure est lente puisque la cadence de l'image est limitée par le balayage mécanique de la tête de tir.

Par ailleurs, avant de savoir faire une image avec des échographes en mode "B", on a utilisé des échographes en mode "A" pour réaliser soit la mesure de distance, par exemple en échographie oculaire, soit la mesure de l'ouverture des valves mitrales, soit la mesure et le repérage de l'axe médian du cerveau, soit encore le repérage de la position d'une génératrice osseuse, en vue d'un traitement comme décrit dans le document précédent de TECHNOMED FR-A-2.660.186 = US-A-5.235.981.

Cependant, un échographe en mode A n'a jamais été utilisé au moment de la thérapie et encore moins pour la surveillance de la position d'un patient pendant une thérapie.

Encore, pour s'assurer de la position d'un patient pendant la thérapie, il a été proposé d'utiliser des marques lumineuses (FR-A-2.663.529 au nom de TECHNOMED INTERNATIONAL; US-A-4.132.900 au nom de W.E. Smith ; DE-A-2.361.155 au nom de C. Lescrenier et EP-A-0.260.550 au nom de SIEMENS A.G.).

Cependant, l'emploi de marques lumineuses n'est évidemment pas utilisable dans certains cas comme, par exemple, dans le cas de traitement endocavitaire.

Ainsi, la présente invention a pour but de résoudre le nouveau problème technique consistant en la fourniture d'une solution permettant de réaliser une surveillance sûre et fiable de la position d'un patient pendant une thérapie. Avantageusement, cette solution doit être particulièrement simple.

La présente invention a encore pour but de résoudre le nouveau problème technique consistant en la fourniture d'une solution permettant de réaliser une surveillance automatique de la position d'un patient pendant une thérapie de manière sûre, fiable et particulièrement simple.

La présente invention a encore pour but de résoudre le nouveau problème technique consistant en la fourniture d'une solution permettant de réaliser non seulement la surveillance de la position d'un patient pendant une thérapie, mais également de réaliser une correction et un asservissement de la position soit du patient, soit de l'appareil de thérapie pendant la thérapie, pour que cette thérapie soit correctement réalisée pendant toute sa durée. De préférence, cette solution doit également permettre de réaliser un asservissement en temps réel d'une sonde de thérapie sur un contour spéculaire prédéterminé, ceci indépendamment de tout autre système d'imagerie conventionnelle.

La présente invention a encore pour but de résoudre les nouveaux problèmes techniques énoncés ci-dessus en fournissant une solution qui permette également d'alerter le praticien et/ou le personnel soignant au cours de la thérapie en cas de changement par inadvertance de la position du patient.

L'invention permet de résoudre simultanément tous les problèmes techniques ci-dessus énoncés de manière sûre et fiable, utilisable à l'échelle industrielle et médicale.

Ainsi, selon un premier aspect, la présente invention concerne l'utilisation d'un échographe en mode A pour la surveillance de la position d'un patient pendant une séance de thérapie.

Dans le cadre de cette utilisation, on peut prévoir avantageusement de réaliser une correction du pointage de l'appareil de thérapie en fonction des mouvements détectés sur cet échographe de mode A.

Également, on peut prévoir d'utiliser cet échographe en mode A pour interrompre la thérapie si les mouvements détectés sont supérieurs à une limite prédéfinie qui peut être fonction de la marge de sécurité.

Selon un mode de réalisation avantageux, l'invention concerne également l'utilisation d'un échographe de type A pour l'asservissement en temps réel d'un dispositif de thérapie, par exemple comprenant une sonde de thérapie, sur un contour spéculaire prédéterminé, avantageusement indépendamment de tout autre système d'imagerie conventionnelle, ce qui est particulièrement simple.

Par exemple, dans le cas d'un traitement de la prostate, il est possible d'asservir le dispositif de thérapie, par exemple comprenant au moins un transducteur de thérapie, sur l'écho A de la paroi rectale, et ceci indépendamment du système d'imagerie. Ceci peut permettre de réaliser des lésions situées à une distance précise, qui peut par exemple être à 3 mm du rectum, pour un traitement correct. De manière similaire, on peut également prévoir, dans le cadre du traitement de la thyroïde, d'asservir le dispositif de thérapie en temps réel sur l'écho de la trachée.

Pour cette thérapie, on pourra utiliser de préférence un appareil de thérapie par ultrasons focalisés.

Selon un deuxième aspect, la présente invention couvre aussi un procédé de surveillance de la position d'un patient, en particulier d'un organe de celui-ci à traiter, pendant une séance de thérapie, comprenant :
a) la prévision d'un transducteur de surveillance ultrasonore en mode A en contact acoustique avec le patient à surveiller, en particulier avec un organe à traiter, ce transducteur étant dans une position connue, par exemple fixé à un bâti, en particulier le support du patient;
b) l'excitation de ce transducteur de surveillance ultrasonore de mode A par un signal impulsionnel court, par exemple entre les tirs de thérapie ;
c) la réception des échos de ce signal sur le même transducteur;
d) la transformation des échos reçus en signal électrique;
e) le repérage sur les échos de ce signal de la position ou de la forme d'une structure caractéristique de l'écho d'un tissu du patient à surveiller;
f) la comparaison de la position ou de la forme de cette structure caractéristique de l'écho d'un tissu du patient à surveiller avec une position ou la forme d'un signal d'écho de référence de ce tissu du patient à surveiller; et
g) lorsque le résultat de cette comparaison résulte en une modification de la position ou de la forme du signal d'écho reçu par rapport à la position ou forme du signal d'écho de référence, de transmettre cette information à un dispositif de commande approprié.

Selon un premier mode de réalisation avantageux, ce dispositif de commande déclenche une alarme lorsque l'information transmise contient une modification de la position de la forme du signal d'écho reçu, ou même l'absence de ce signal d'écho reçu, par rapport au signal d'écho de référence.

Selon un autre mode de réalisation avantageux de l'invention, le dispositif de commande réalise un asservissement en temps réel de la position de l'appareil de thérapie en fonction des déplacements du patient, en particulier de l'organe à traiter, de préférence sur un contour spéculaire prédéterminé du patient, tel que par exemple la paroi rectale lors d'un traitement de la prostate ou la trachée lors du traitement de la thyroïde.

Selon un autre mode de réalisation avantageux, le transducteur de surveillance ultrasonore en mode A peut être lié mécaniquement au dispositif de thérapie.

Selon un autre mode de réalisation avantageux, le dispositif de thérapie comprend au moins un transducteur ultrasonore de thérapie permettant une thérapie par ultrasons focalisés.

Selon une variante de réalisation préférée, ce transducteur ultrasonore de thérapie est disposé à l'intérieur d'un ballonnet rempli d'un fluide de couplage acoustique.

Selon un mode de réalisation avantageux, de préférence, dans ce cas, l'écho de signal de référence peut être constitué par la distance théorique séparant une interface à fort pouvoir échogène par exemple l'interface en contact avec la paroi du ballonnet, du transducteur ultrasonore de thérapie et la comparaison précitée consistera à comparer la distance réelle de l'écho correspondant à cette interface, à la distance de référence de cette interface relativement au transducteur de thérapie.

Selon une première variante de réalisation, si la distance réelle de l'écho est plus petite que la distance de référence, on commandera un éloignement du transducteur de thérapie par rapport au patient, en particulier un organe à traiter, tandis que selon une autre variante, si la distance réelle de l'écho est plus grande que la distance théorique, on rapprochera le transducteur de thérapie par rapport au patient, en particulier par rapport à l'organe à traiter.

Selon un autre mode de réalisation avantageux, le transducteur ultrasonore de thérapie est un transducteur ultrasonore de thérapie focalisé intégré dans une sonde endocavitaire. Cette sonde endocavitaire est de préférence une sonde endocavitaire rectale, en permettant ainsi de traiter les tumeurs bénignes ou malignes en particulier de la prostate ou de la thyroïde, notamment les cancers.

D'autres modes de réalisation avantageux apparaîtront également à partir de la description suivante relativement à l'appareil de thérapie, ainsi qu'à partir des exemples en référence aux dessins qui font partie intégrante de l'invention et donc de la présente description.

Selon un troisième aspect, la présente invention couvre également un appareil de thérapie comprenant un dispositif de thérapie comprenant au moins un transducteur ultrasonore de thérapie et au moins un transducteur ultrasonore de surveillance, caractérisé en ce que le transducteur ultrasonore de surveillance est relié à un circuit électronique de type mode A, ledit appareil comprenant également des moyens pour faire émettre un signal court au transducteur ultrasonore de surveillance, pour recevoir les échos de ce signal sur ledit transducteur de surveillance, des moyens de comparaison de l'écho reçu par rapport à un écho de référence et des moyens de transmission du résultat de cette comparaison à un dispositif de commande.

Selon un mode de réalisation avantageux, ce dispositif de commande déclenche une alarme.

Selon un autre mode de réalisation, le dispositif de commande réalise un asservissement en temps réel de la position du transducteur de thérapie relativement au patient, en particulier un organe à traiter.

Selon un autre mode de réalisation, le transducteur ultrasonore de thérapie est de type focalisé.

Selon un autre mode de réalisation, le transducteur ultrasonore de thérapie, ainsi que le transducteur de surveillance en mode A, sont intégrés à une sonde endocavitaire, en particulier à une sonde endocavitaire rectale.

Selon un autre mode de réalisation, le transducteur de thérapie précité de type focalisé est relié à des moyens de délivrance d'un signal électronique réalisant une focalisation variable. De préférence, le dispositif de commande précité réalise en un asservissement de la distance focale du transducteur de thérapie en fonction des mouvements du patient pendant la thérapie.

Selon un mode de réalisation, l'appareil de thérapie comprend des moyens pour réaliser un enregistrement des échos en continu du transducteur en mode A. Le circuit électronique de type mode A peut comprendre notamment un échographe travaillant en mode A.

D'autres caractéristiques de l'appareil selon l'invention apparaîtront également clairement à partir de la description du procédé qui précède, ainsi que de la description suivante faite en référence aux exemples de réalisation de l'invention, ainsi qu'à partir des dessins, qui font partie intégrante de l'invention et donc de la présente description.

On comprend que l'invention permet de réaliser une surveillance de la position d'un patient pendant une thérapie d'une manière simple, sûre et fiable, et permet ainsi de résoudre les problèmes techniques précédemment énoncés, ainsi que de fournir d'autres avantages techniques qui apparaîtront également à l'homme de l'art.

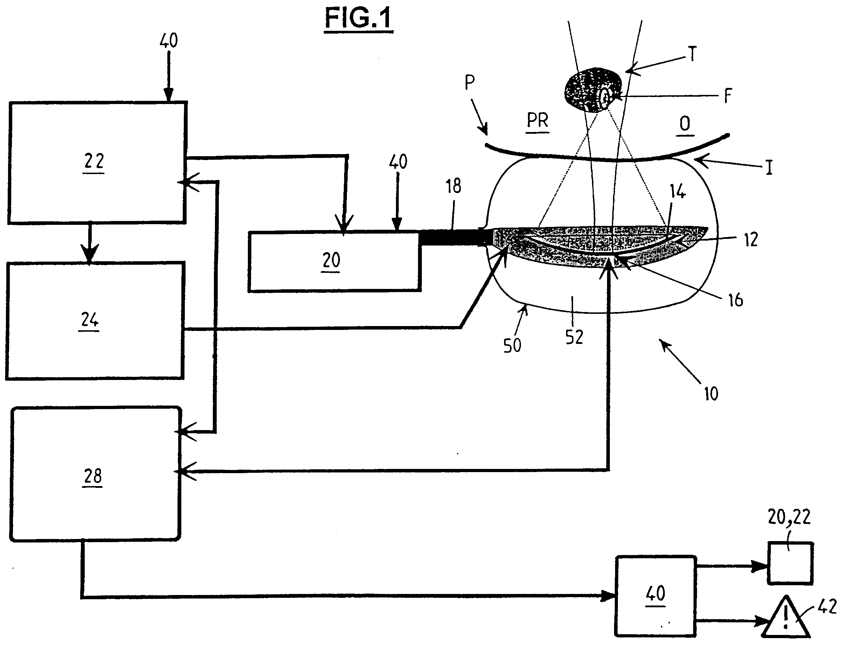

Dans les dessins
- la figure 1 représente un schéma de principe d'un premier mode de réalisation d'un appareil de thérapie selon la présente invention, représentant en coupe transversale longitudinale le dispositif de thérapie proprement dit sous forme d'une sonde endocavitaire ici rectale avec un élément transducteur de surveillance en mode A, ainsi que les organes de commande et de détection essentiels ;

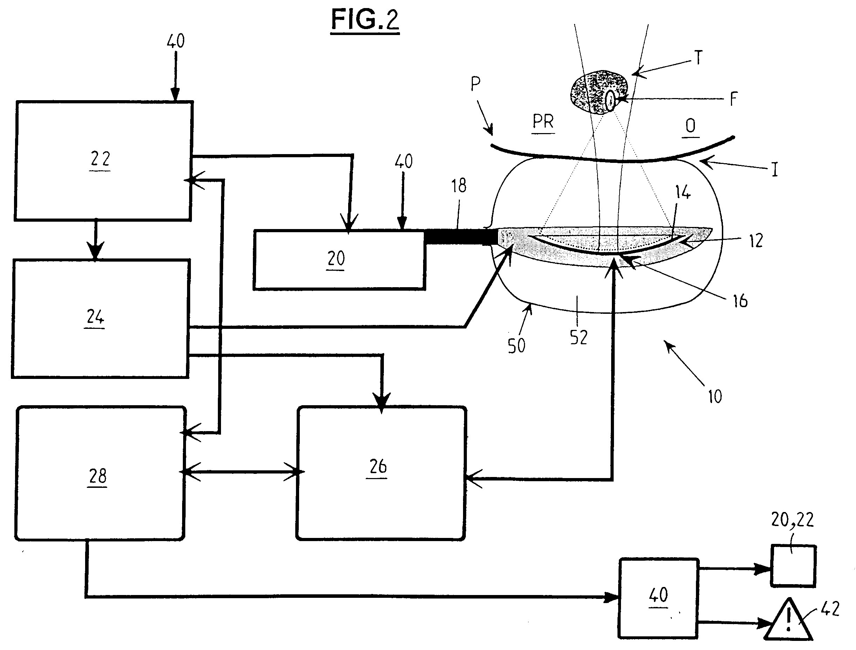

- la figure 2, représente une variation du mode de réalisation du premier mode de réalisation de la figure 1 ;

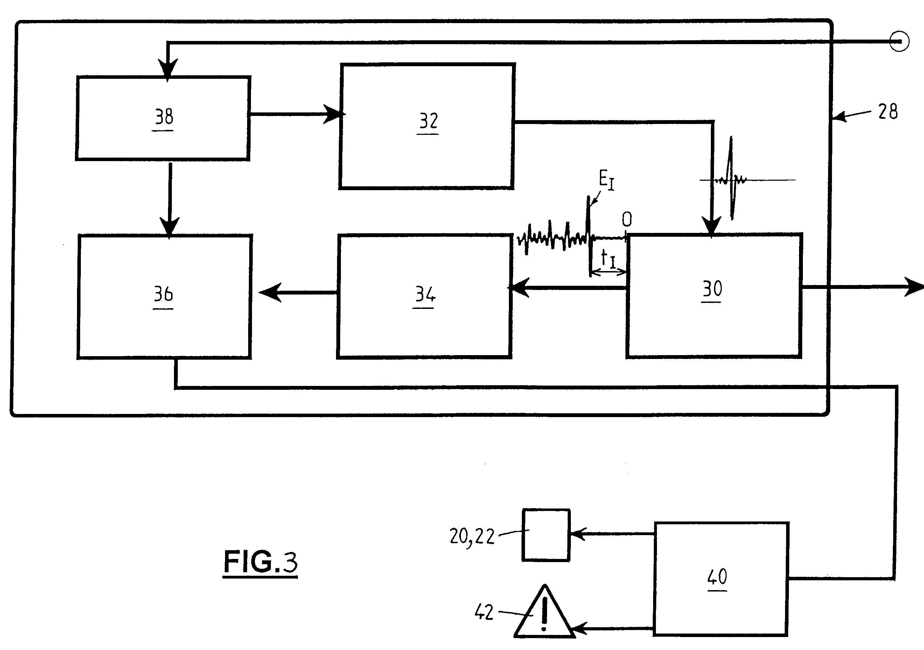

- la figure 3 représente plus particulièrement le détail du bloc du détecteur de mouvements;

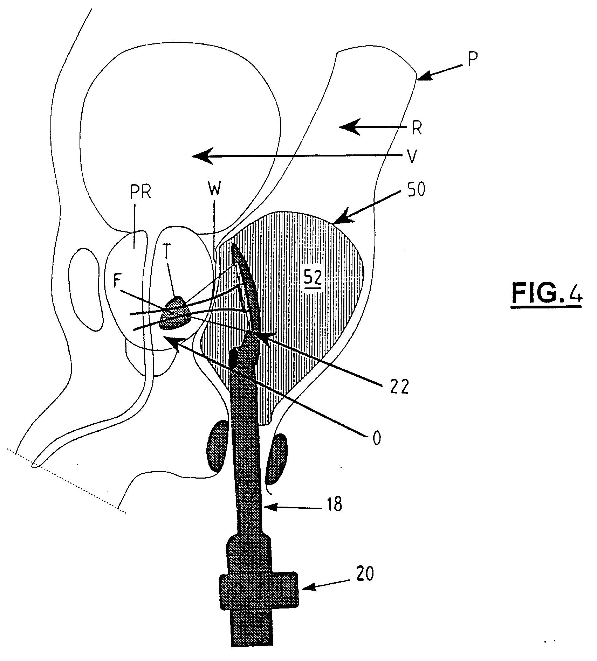

- la figure 4 représente un deuxième mode de réalisation d'un appareil de thérapie selon la présente invention, ici comprenant un dispositif de thérapie sous forme d'une sonde endocavitaire rectale, pour le traitement de la prostate, en position de travail, dans un ballonnet rempli d'un fluide de couplage acoustique avec le ballonnet en contact avec l'organe à traiter ;

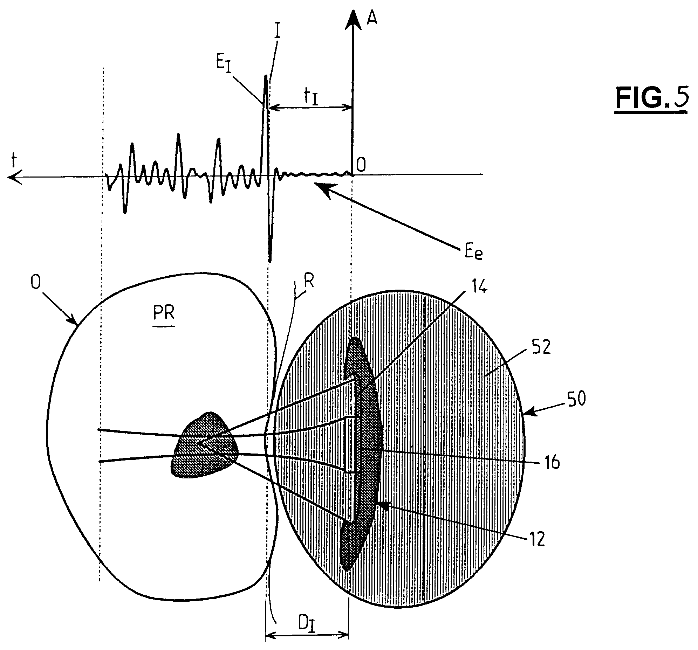

- la figure 5 représente schématiquement de manière agrandie le dispositif de thérapie couplé au transducteur de surveillance ultrasonore en mode A représenté à la figure 4, ainsi que le signal d'écho reçu par le transducteur en mode A suite à une émission d'un signal impulsionnel court, permettant d'observer la structure et la forme du pic d'écho correspondant à l'interface à surveiller, ici la paroi rectale ; et

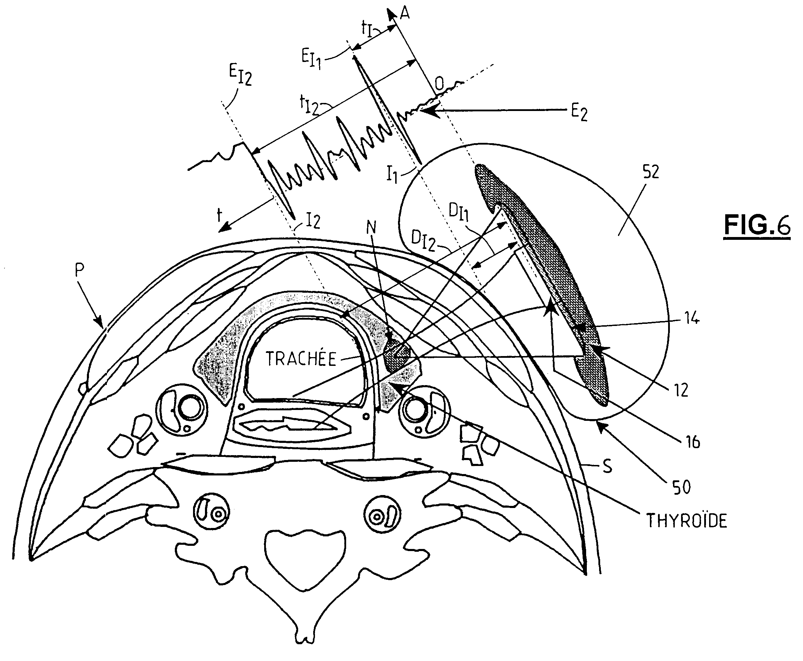

- la figure 6 représente un troisième mode de réalisation d'un appareil de thérapie selon la présente invention, le dispositif de thérapie étant sous forme d'une sonde extracorporelle en coupelle focalisante, ici pour le traitement de la thyroïde selon une coupe horizontale passant par la thyroïde.

En référence aux figures 1 à 4, un appareil de thérapie selon la présente invention est représenté par le numéro de référence générale 10. Cet appareil de thérapie comprend un dispositif de thérapie proprement dit portant le numéro de référence générale 12, comprenant un transducteur de thérapie 14 ici sous forme d'une coupelle naturellement focalisante ainsi qu'au moins un transducteur de surveillance ultrasonore 16 relié à un circuit électronique de type mode A. Il est bien compréhensible à un homme de l'art que le transducteur 16 peut faire partie d'un échographe travaillant en mode A.

Selon un mode de réalisation préféré, la partie active du transducteur de surveillance ultrasonore 16 fait partie intégrante du transducteur de thérapie 14 et ne constitue donc pas un transducteur distinct de celui-ci, bien qu'une telle variante soit possible.

L'élément de surveillance pourra être situé au centre du transducteur sur son axe de symétrie.

Le transducteur de surveillance peut être obtenu par partition de la couche métallique qui est déposée sur le matériau piézo-électrique formant transducteur de thérapie pour l'établissement de son excitation électrique. Cette méthode permet d'obtenir facilement 2 éléments piézo-acoustiques commandables séparément.

La partition de la couche de métallisation pourra être conçue de telle sorte que l'élément de surveillance ait une surface minimale de manière à ne diminuer que peu la surface émissive de l'élément utilisé pour la thérapie.

Selon un mode de réalisation avantageux, le dispositif de thérapie 12 peut prendre la forme d'une sonde 18 endocavitaire du type qui est décrit dans le document WO 92/15253, qui est incorporée ici par référence.

En particulier, et selon également ce document antérieur, la partie arrière de la sonde endocavitaire 18 peut comprendre un système de moteur 20 permettant une commande en translation et/ou en rotation de la sonde endocavitaire 18, permettant de positionner précisément la sonde endocavitaire en regard d'une cible T à traiter, telle qu'une tumeur disposée à l'intérieur d'un organe O par exemple la prostate PR défini par une enveloppe formant interface I d'un patient P. Ces références se retrouveront également dans les modes de réalisation des figures 4 à 6.

L'appareil de thérapie comprend également une centrale de commande 22 permettant non seulement de commander les moyens moteurs 20 de translation et/ou de rotation de la sonde endocavitaire 18, mais également un générateur de signal électronique 24, commandant le fonctionnement du transducteur de thérapie 14 et/ou le transducteur de surveillance par l'intermédiaire d'un dispositif électronique détecteur de mouvement 28 dont la structure est décrite plus en détail à la figure 3. On peut aussi prévoir la présence d'un circuit de commutation 26 comme représenté à la figure 2 (disposé avec le dispositif 28). Le dispositif détecteur de mouvements représenté à la figure 3 constitue une électronique de traitement qui comporte deux sous-ensembles reliés au transducteur de surveillance 16 par l'intermédiaire d'un dispositif aiguilleur 30 relié d'une part au générateur d'impulsion 32 et, d'autre part, à un dispositif amplificateur 34 combiné à un dispositif détecteur 36. Ces deux sous-ensembles sont eux-mêmes synchronisés par un dispositif de synchronisation 38 sur un signal de déclenchement délivré par la centrale de commande 22. Le dispositif amplificateur 34 - détecteur 36 analyse l'écho reçu par le transducteur de surveillance 16 et génère un signal de commande d'un dispositif de commande 40 qui peut être soit une alarme, soit des moyens de commande, des moyens de déplacement 20 en translation et/ou en rotation de la sonde endocavitaire 18 éventuellement par la centrale de commande 22.

Le fonctionnement de l'appareil de traitement selon la présente invention apparaîtra clairement des deux exemples de traitement des figures 4 à 6.

En référence aux figures 4 à 6, on observera tout d'abord que le transducteur de thérapie 14 émet un faisceau focalisé à la zone focale F, ici symbolisé en forme de triangle qui, pour une bonne thérapie, doit se trouver sur ou dans la zone cible à traiter, telle qu'une tumeur T à l'intérieur d'un organe O qui peut être, par exemple, la prostate comme cela sera décrit en référence à la figure 4 ou la thyroïde comme cela sera décrit en référence à la figure 6.

Dans le mode de réalisation représenté ici dans les figures 1 à 6, le transducteur 16 de surveillance en mode A est intégré dans le transducteur de thérapie 14, par exemple en occupant la partie centrale de celui-ci, comme cela se voit bien aux figures 2, 4 à 6.

Selon un mode de réalisation avantageux, le transducteur de thérapie 14, intégrant le transducteur 16 de surveillance, peut être fabriqué en matériau composite comme décrit dans le document précédent de TECHNOMED INTERNATIONAL FR-A-2 679 125 qui est incorporé ici par référence.

On peut aussi, comme cela est représenté sur la figure 2, utiliser l'élément central du transducteur à la fois pour la surveillance et pour la thérapie. Dans ce cas, on le reliera successivement, par l'intermédiaire d'un circuit de commutation 26, au générateur de signal électronique de puissance 24 ou au dispositif de détection du mouvement 28.

Le même transducteur central peut être utilisé également pour caractériser le tissu cible, avant, pendant ou après le traitement. Dans ce mode, les échos de retour sont analysés afin de déterminer la structure du tissu traité ou l'effet du traitement lui-même.

Le transducteur de surveillance 16 émet un signal en mode A dont la forme du faisceau est représentée sur chaque figure 1, 2, 4, 5 et 6. Il est préféré dans le cadre de l'invention que le transducteur de surveillance 16 en mode A ait son faisceau qui couvre le point focal F pour permettre également une surveillance de la zone focale pendant le traitement.

Cependant, dans le cadre de la surveillance de la position du patient, en particulier de l'organe O, il n'est pas absolument nécessaire que le faisceau du transducteur de surveillance 16 en mode A passe par la zone focale, puisque le but essentiel de ce transducteur de surveillance est de surveiller la position d'une surface fortement échogène, qui sera généralement constituée par l'interface I de la trachée du patient P comme cela sera décrit en référence à la figure 6, ou la paroi de l'organe O à traiter, par exemple la prostate PR comme montré aux figures 1, 2, 4 et 5, ces interfaces étant fortement échogènes.

On observera ici que le dispositif de thérapie est de préférence inséré à l'intérieur d'une membrane 50 remplie d'un liquide de couplage acoustique 52, par exemple de l'eau, qui permet de transmettre les ondes acoustiques jusqu'à la cible, par exemple la tumeur T dans l'organe O, ici aux figures 4 et 5 la prostate PR.

Dans le cadre des figures 4 et 5, qui concernent plus particulièrement le traitement de la prostate, l'interface à surveiller I est la paroi rectale R car par l'emploi d'ultrasons focalisés, le point focal F du transducteur de thérapie 14 focalisé est positionné très près de la paroi rectale W. Il y a alors un risque pour le patient P, puisqu'un positionnement erroné du dispositif de thérapie 12 peut entraîner la destruction de la paroi rectale W, ce qui a naturellement des conséquences graves. Il est donc très important de surveiller en permanence la position de la paroi rectale par rapport au transducteur de thérapie 14.

Pour réaliser cette surveillance, le transducteur de surveillance 16, qui est ici la partie centrale du transducteur de thérapie 14, émet une impulsion courte, par exemple à la fréquence de 2,25 MHz pendant 1 µs, par l'intermédiaire du générateur d'impulsions 32 dont l'écho est recueilli par le dispositif détecteur de mouvements 28 en étant traité par le dispositif amplificateur 34, détecteur 36 qui analyse l'écho et le compare avec un écho de référence.

Pour comprendre comment se passe cette comparaison, à la figure 5, on a représenté la forme du signal d'écho en fonction du temps T en abscisse avec son amplitude A en ordonnées. Le signal d'écho E lorsqu'il est émis à l'instant 0 du transducteur de surveillance 16 traverse une zone peu échogène Eₑ, due au liquide de couplage, telle que de l'eau, puis rencontre l'interface I constituée par la paroi rectale et ici la paroi ou coque de la prostate PR constituant ici l'organe O à traiter. Cette interface I constituée par la paroi rectale W est fortement échogène et résulte à un écho référencé E_{I}, qui est l'écho que l'on va surveiller dans le cadre de l'invention. On observe que cet écho est rencontré en un temps t_{I} depuis la génération du signal qui correspond au parcours de la distance D_{I} entre le transducteur de surveillance 16 et la paroi rectale W dans la zone de surveillance du transducteur de surveillance 16.

Dans le cadre de l'invention, on comprend que lorsque le patient va bouger, il va également, dans le cadre des figures 4 et 5, faire bouger l'organe O, ici la prostate PR, ce qui va modifier la distance D_{I} entre le transducteur de surveillance 16 et la paroi rectale W.

Dans le cadre de l'invention, selon une première variante de réalisation, on peut simplement comparer l'instant t_{I} de l'écho E_{I} reçu avec un écho de référence qui peut être l'écho reçu juste avant ou en début de traitement, ou bien comparer la distance parcourue par l'écho de retour avec la distance relevée par les moyens de repérage préalablement au traitement. Lorsque ce temps t_{I} devient différent du temps de l'écho de référence, il en résulte que la distance D_{I} a changé par rapport à la distance entre le transducteur de surveillance 16 et la paroi rectale mesurée initialement, ce qui caractérise un mouvement du patient. Dans ce cas, le dispositif de détection et d'analyse 36 peut délivrer au dispositif de commande 40 un ordre de commande d'une alarme et/ou un ordre de commande de déplacement des dispositifs 20 de déplacement en translation et/ou en rotation du dispositif de thérapie 12 incorporant le transducteur de surveillance 16 pour que l'écho de l'interface I apparaisse à nouveau à un temps t_{I} identique ou voisin du temps d'apparition de l'écho de référence.

On constate ainsi que l'on peut prévoir de déclencher une alarme ou un ordre de déplacement du transducteur de thérapie 16 seulement lorsque le temps t_{I} de l'écho d'interface E_{I} est inférieur ou supérieur à une valeur prédéterminée de temps de l'écho de référence.

On constate qu'ainsi avec l'invention il est aisé d'automatiser la surveillance des mouvements du patient ainsi que de la rectification de la position du dispositif de thérapie 12 pendant le traitement.

On peut également prévoir non seulement de calculer le temps t_{I} de l'écho de l'interface E_{I} mais également la distance D_{I} qui lui correspond en raison de la vitesse connue sensiblement constante du son dans le liquide de couplage acoustique et dans les tissus, ainsi que d'afficher cette distance D_{I}, ainsi que le temps t_{I} sur un écran d'échographe, ce qui permet au praticien de surveiller le traitement avec une plus grande sécurité.

En référence à la figure 6, on a représenté un autre mode de réalisation de l'invention dans le cadre du traitement de nodules N de la thyroïde. On a utilisé ici les mêmes chiffres et signes de référence qu'aux figures 4 et 5 pour les éléments identiques.

Ici, l'interface très importante à surveiller est l'interface de la trachée dénommée ici interface I₂. Cette interface de la trachée génère également un écho fort dénommé *E*_{*I2*}, que l'on voit bien sur la forme du signal d'écho représentée à la figure 6 pour une meilleure compréhension. Avant cette interface de la trachée I₂, il existait naturellement une première interface I₁ constituée par la peau S du patient P mais ici, cette interface qui génère par ailleurs un premier écho fort *E*_{*I1*} est de faible importance par rapport à l'interface de la trachée I₂.

Dans le cas du traitement de la thyroïde, on se servira donc, le temps d'apparition de l'écho E_{I}, de l'interface I₂ référencée t_{I2} qui correspond à la distance D_{I2}, entre le transducteur de surveillance 16 et l'interface I₂ de la trachée.

La surveillance s'opère de la même manière que dans le cas des figures 4 et 5, les modifications de ce temps d'apparition t_{I2}, de l'écho E_{I2}, par rapport à un temps d'apparition de référence, permettant de déclencher un signal d'alarme sur le dispositif de commande 40 et/ou des ordres de modification de la position du dispositif de thérapie 12 par l'intermédiaire de la centrale de commande 22 et des dispositifs de déplacement en translation et/ou en rotation 20.

L'invention permet donc de résoudre les problèmes techniques précédemment énoncés, et d'aboutir à tous les avantages techniques qui en résultent et qui apparaissent également clairement à un homme de l'art.

Bien entendu, l'invention comprend tous les moyens constituant des équivalents techniques des moyens décrits ainsi que leurs diverses combinaisons.

Par exemple, on peut également prévoir une émission d'un signal continu par le transducteur de surveillance 16 en mode A ainsi qu'un enregistrement continu des échos, ce qui peut fournir un contrôle visuel des mouvements de déplacement du patient P.

D'autre part, la présente invention couvre toute caractéristique qui apparaît être nouvelle vis-à-vis d'un état de la technique quelconque à partir de la description incorporant les figures 1 à 6 et qui en font partie intégrante, ainsi que des revendications.

## Revendications

1. Utilisation d'un échographe en mode A pour la surveillance de la position d'un patient pendant une séance de thérapie.

2. Utilisation selon la revendication 1, caractérisée en ce qu'une correction du pointage de l'appareil de thérapie en fonction des mouvements détectés sur cet échographe de mode A est réalisée.

3. Utilisation selon la revendication 1 ou 2, caractérisée en ce que l'échographe en mode A est utilisé pour interrompre la thérapie si les mouvements détectés sont supérieurs à une limite prédéfinie qui peut être fonction de la marge de sécurité.

4. Utilisation selon l'une des revendications 1 à 3, caractérisée en ce que l'échographe en mode A est utilisé pour réaliser un asservissement en temps réel d'un appareil de thérapie pouvant par exemple comprendre une sonde de thérapie, sur un contour spéculaire prédéterminé, de préférence indépendamment de tout autre système d'imagerie conventionnelle.

5. Procédé de surveillance de la position d'un patient, en particulier d'un organe de celui-ci à traiter, pendant une séance de thérapie, comprenant :
a) la prévision d'un transducteur de surveillance ultrasonore en mode A
en contact acoustique avec le patient à surveiller, en particulier avec un organe à traiter, ce transducteur étant dans une position connue, par exemple fixé à un bâti, en particulier le support du patient ;
b) l'excitation de ce transducteur de surveillance ultrasonore de mode A par un signal impulsionnel court, par exemple entre les tirs de thérapie ;
c) la réception des échos de ce signal sur le même transducteur
d) la transformation des échos reçus en signal électrique ;
e) le repérage sur les échos de ce signal de la position ou de la forme d'une structure caractéristique de l'écho d'un tissu du patient à surveiller ;
f) la comparaison de la position ou de la forme de cette structure caractéristique de l'écho d'un tissu du patient à surveiller avec une position ou la forme d'un signal d'écho de référence de ce tissu du patient à surveiller; et
g) lorsque le résultat de cette comparaison résulte en une modification de la position ou de la forme du signal d'écho reçu par rapport à la position ou forme du signal d'écho de référence, de transmettre cette information à un dispositif de commande approprié.

6. Procédé selon la revendication 5, caractérisé en ce que le dispositif de commande précité déclenche une alarme lorsque l'information transmise contient une modification de la position de la forme du signal d'écho reçu, ou même l'absence de ce signal d'écho reçu, par rapport au signal d'écho de référence.

7. Procédé selon la revendication 5 ou 6, caractérisé en ce que le dispositif de commande réalise un asservissement en temps réel de la position du dispositif de thérapie en fonction des déplacements du patient, en particulier de l'organe à traiter, de préférence sur un contour spéculaire prédéterminé du patient, tel que la paroi rectale lors d'un traitement de la prostate, ou la trachée lors d'un traitement de la thyroïde.

8. Procédé selon l'une des revendications 5 à 7, caractérisé en ce que le transducteur de surveillance ultrasonore en mode A est lié mécaniquement au dispositif de thérapie.

9. Procédé selon l'une des revendications 5 à 8, caractérisé en ce que le dispositif de thérapie comprend au moins un transducteur ultrasonore de thérapie permettant une thérapie par ultrasons focalisés.

10. Procédé selon l'une des revendications 5 à 9, caractérisé en ce que le transducteur ultrasonore de thérapie est disposé à l'intérieur d'un ballonnet rempli d'un fluide de couplage acoustique.

11. Procédé selon l'une des revendications 5 *à* 10, caractérisé en ce que l'écho de signal de référence précité est constitué par la distance théorique séparant un interface à fort pouvoir échogène, par exemple l'interface en contact avec la paroi du ballonnet, du transducteur ultrasonore de thérapie et la comparaison précitée consistera à comparer la distance réelle de l'écho correspondant à cet interface à la distance de référence de cet interface relativement au transducteur de thérapie.

12. Procédé selon la revendication 11, caractérisé en ce que si la distance réelle est plus petite que la distance de référence, on commandera un éloignement du transducteur de thérapie par rapport au patient, en particulier un organe à traiter, tandis que si la distance réelle est plus grande que la distance théorique, on rapprochera le transducteur de thérapie par rapport au patient, en particulier par rapport à l'organe à traiter.

13. Procédé selon l'une des revendications 5 à 12, caractérisé en ce que le transducteur ultrasonore de thérapie est un transducteur ultrasonore de thérapie focalisé intégré dans une sonde endocavitaire, de préférence rectale, en permettant ainsi de traiter les tumeurs bénignes ou malignes en particulier de la prostate ou de la thyroïde, notamment les cancers.

14. Appareil de thérapie (10) comprenant un dispositif de thérapie (12) comprenant au moins un transducteur ultrasonore de thérapie (14) et au moins un transducteur ultrasonore de surveillance (16), caractérisé en ce que le transducteur ultrasonore de surveillance est relié à un circuit électronique de type A, par exemple en faisant partie d'un échographe en mode A, ledit appareil comprenant également des moyens (24, 26, 28) pour faire émettre un signal court au transducteur ultrasonore en mode A, pour recevoir les échos de ce signal sur ledit transducteur en mode A, des moyens de comparaison (26, 28) de l'écho reçu par rapport à un écho de référence et des moyens (28) de transmission du résultat de cette comparaison à un dispositif de commande (40).

15. Appareil selon la revendication 14, caractérisé en ce que le dispositif de commande (40) déclenche une alarme (42).

16. Appareil selon la revendication 14, caractérisé en ce que le dispositif de commande (40) réalise un asservissement en temps réel de la position du transducteur de thérapie (14) relativement au patient (P), en particulier un organe à traiter (O), de préférence le dispositif de commande réalise un asservissement en temps réel de la position du dispositif de thérapie sur un contour spéculaire prédéterminé du patient, tel que la paroi rectale dans le cadre du traitement de la prostate ou la trachée dans le cadre du traitement de la thyroïde.

17. Appareil selon l'une des revendications 14 à 16, caractérisé en ce que le transducteur ultrasonore de thérapie (16) est de type focalisé.

18. Appareil selon l'une des revendications 14 à 17, caractérisé en ce que le transducteur ultrasonore de thérapie (14), ainsi que le transducteur en mode A (16), sont intégrés à une sonde endocavitaire (18), en particulier à une sonde endocavitaire rectale.

19. Appareil selon l'une des revendications 14 à 18, caractérisé en ce que le transducteur de thérapie (14) précité de type focalisé est relié à des moyens de délivrance d'un signal électronique réalisant une focalisation variable.

20. Appareil selon la revendication 19, caractérisé en ce que le dispositif de commande précité (40) réalise un asservissement de la distance focale du transducteur de thérapie (14) en fonction des mouvements du patient (P) pendant la thérapie.

21. Appareil selon l'une des revendications 14 à 20, caractérisé en ce que l'appareil de thérapie comprend des moyens (26, 28) pour réaliser un enregistrement des échos en continu de transducteur de surveillance en mode A (16).

22. Appareil selon l'une des revendications 14 à 21, caractérisé en ce que le transducteur de surveillance ultrasonore (16) est lié mécaniquement au dispositif de thérapie (12).

23. Appareil selon l'une des revendications 14 à 22, caractérisé en ce que le dispositif de thérapie comprend au moins un transducteur ultrasonore de thérapie (14) permettant une thérapie par ultrasons focalisés, le transducteur ultrasonore de thérapie étant de préférence disposé à l'intérieur d'un ballonnet (50) rempli d'un fluide de couplage acoustique (52).

24. Appareil selon la revendication 23, caractérisé en ce que l'écho de référence est constitué par la distance théorique (D_{I}; D_{I1}, ou D_{I2}) séparant une interface à fort pouvoir échogène, par exemple l'interface (I ou I₁) en contact avec la paroi du ballonnet, du transducteur ultrasonore de thérapie (14) et les moyens de comparaison précitée comparent la distance réelle de l'écho correspondant à cette interface à la distance de référence de cette interface relativement au transducteur de thérapie (14), et le dispositif de commande (40) commande soit un éloignement du transducteur de thérapie (14) par rapport au patient, en particulier un organe à traiter (O), si la distance réelle de l'écho est plus petite que la distance de référence, soit un rapprochement du transducteur de thérapie (14) par rapport au patient (P), en particulier par rapport à l'organe à traiter (O), si la distance réelle de l'écho est plus grande que la distance théorique.

25. Appareil selon l'une des revendications 14 à 24, caractérisé en ce qu'il s'agit d'un appareil de traitement des tumeurs bénignes ou malignes, en particulier de la prostate ou de la thyroïde, notamment les cancers.
